# EUROPEAN PATENT SPECIFICATION

(11) **EP 4 551 136 B1**
(45) Date of publication and mention of the grant of the patent: **25.11.2026**
(21) Application number: 22751521.0
(22) Date of filing: 07.07.2022
(51) Int. Cl.: A61B 17/3207

(54) **ENDOVASCULAR APPARATUS FOR TREATING VESSEL INTIMA**
ENDOVASKULÄRE VORRICHTUNG ZUR BEHANDLUNG VON GEFÄSSINTIMA
APPAREIL ENDOVASCULAIRE POUR LE TRAITEMENT DE L'INTIMA VASCULAIRE

(43) Date of publication of application: 14.05.2025
(73) Proprietor: Bard Peripheral Vascular, Inc., Franklin Lakes, NJ 07417 (US)
(72) Inventor: VAN LIERE, Chad, Phoenix, AZ 85042 (US)
(74) Representative: Rondano, Davide
(86) International application number: PCT/US2022/036369
(87) International publication number: WO 2024/010583

(56) References cited:
- EP-A1- 0 117 519
- US-A1- 2002 010 487
- US-A1- 2012 035 594
- US-A1- 2017 238 960

## Description

### BACKGROUND

Proposals exist for treating a diseased vessel using a rotating endovascular device for ablating or debriding tissue along the interior of the vessel, or intima. Existing approaches typically involve rotating a single-bladed cutter in one direction only. Such a cutter may successfully ablate the tissue (e.g., the endothelial layer) directly underneath the point of contact, but other local tissue may deform circumferentially upon being engaged and reduce the effect of the intended ablation or debridement.

Additionally, the only adjustable variable to alter performance is to change the rotational speed of the cutter. If the rotation is too slow, the procedure will take a prohibitively long time and decrease efficacy. If the rotation is too fast, the dynamics can cause unintended damage beyond the intent of the procedure. High speed rotation of the cutter in one direction also induces varying levels of localized torsion in the vessel, depending on tissue dynamics, which may reduce efficacy.

Accordingly, a need is identified for an improved endovascular apparatus for treating vessel intima that overcomes any or all of the foregoing limitations and possibly others that have yet to be identified.

An endovascular apparatus for treating vessel intima according to the preamble of independent claim 1 is known from US 2017/238960 A1.

### SUMMARY

The invention is defined by independent claim 1, with further embodiments defined by the dependent claims.

An object of the disclosure is to provide an apparatus for endovascular use, such as for treating a vessel intima, including a plurality of nested hoops connected to the shaft and adapted to rotate relative to one another, such as to allow for counter-rotation. As a result of the offsetting forces, such rotating hoops virtually eliminate torsional effects from using such an apparatus. Compared to a single blade with one point of contact, a plurality of hoops also establish multiple points of contact with the vessel (e.g., two hoops make four points of contact). The effect of the edges of the nested hoops approaching each other during relative rotation allows for tissue to be dynamically sheared at double the rate of a single cutter. Consequently, the proposed apparatus could rotate with less velocity than a single bladed device (by as much as two to four times) to achieve the same ablating effect. Assuming rotational speeds equivalent to the current approaches, then use of the proposed apparatus would potentially result in half the time to perform a procedure.

According to a first aspect of the disclosure, an apparatus for endovascular use in treating a vessel intima is disclosed. The apparatus comprises a catheter adapted to form a plurality of nested hoops capable of rotating relative to one another in a deployed position.

The plurality of nested hoops comprise a first inner hoop having an outer diameter less than an inner diameter of a second, outer hoop. The catheter further comprises an inner tube connected to the first inner hoop and an outer tube connected to the second inner hoop. The outer tube may coiled, such as to facilitate flexibility and trackability of the catheter through tortuous anatomy.

The inner tube and the outer tube form an annulus for delivery of a treatment agent. The inner hoop and outer hoop may comprise a pair of arcuate members connected to the respective inner tube or outer tube. A distal end of the inner tube may be rotatably connected or coupled to the outer tube, and the inner tube may be connected to a rod for advancing distally to form the inner hoop and outer hoop (such as by moving portions of the respective tubes from a substantially flat position to an outwardly bowed position).

According to a further aspect of the disclosure, an apparatus for endovascular use is provided. The apparatus comprises a catheter including a plurality of hoops, including a first inner hoop having an outer diameter less than an inner diameter of a second, outer hoop, the first, inner hoop being adapted to rotate relative to the second outer hoop.

In one embodiment, the catheter comprises an inner tube connected to the first inner hoop and an outer tube connected to the second inner hoop. The outer tube may coiled. The inner and outer tube may form an annulus for delivery of a treatment agent. A distal end of the inner tube may be rotatably connected or coupled to the outer tube, and the inner tube may be connected to a rod for advancing distally to form the inner hoop and outer hoop (such as by moving portions of the respective tubes from a substantially flat position to an outwardly bowed position).

In this or other embodiments, one or more motors are adapted for causing relative rotation of the plurality of nested hoops. The relative rotation may be counter-rotation. A first hoop of the plurality of hoops is partially received within a portion of a second hoop.

Each hoop may comprise a pair of opposed arcuate members. Each hoop may be generally elliptical.

A further aspect of the disclosure, not forming part of the claimed invention, relates to a method of performing a procedure in a vessel. The method comprises engaging an interior of the vessel with a first device to cause the vessel to assume a generally elliptical cross-section, and rotating a second device within the vessel to engage the interior of the vessel.

In one example, the engaging step comprises engaging the interior of the vessel with a first outer hoop as the first device, and the second device of the rotating step comprises a second inner hoop nested within the first outer hoop. The engaging step may comprise rotating the first outer hoop, such as at a first rotational speed less than a rotational speed of the second inner hoop. The first outer hoop and second inner hoop are capable of being rotated in opposite directions.

### BRIEF DESCRIPTION OF THE DRAWINGS

The above and further advantages of the disclosure may be better understood by referring to the following description in conjunction with the accompanying drawings in which:
FIG. 1 illustrates a perspective view of a device for treating a vessel intima according to a first aspect of the disclosure;
FIG. 2 is a partially cross-sectional perspective view of the device of FIG. 1;
FIG. 3 is a partially cross-sectional side view of the device of FIG. 1;
FIG. 4 is an exploded side view of the device of FIG. 1;
FIG. 5 is a perspective view of the device of FIG. 1 positioned in a vessel;
FIG. 5A is a cross-sectional view taken along line 5A-5A of FIG. 5;
FIG. 6 is a schematic view of a further embodiment of a device for treating a vessel intima;
FIGS. 7-8 illustrate a further embodiment of a device for treating a vessel intima in retracted and deployed positions; and
FIG. 9 illustrates a further embodiment of a device for treating a vessel intima.

The dimensions of some of the elements may be exaggerated relative to other elements for clarity or several physical components may be included in one functional block or element. Further, sometimes reference numerals may be repeated among the drawings to indicate corresponding or analogous elements. Moreover, some of the items depicted in the drawings may be combined into a single function.

### DETAILED DESCRIPTION

In the following detailed description, numerous specific details are set forth to provide a thorough understanding of the present invention. The disclosed embodiments may be practiced without these specific details. In other instances, well-known methods, procedures, components, or structures may not have been described in detail so as not to obscure the present invention.

The principles and operation of the apparatus and methods of the disclosure may be better understood with reference to the drawings and accompanying descriptions. The invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein are for the purpose of description and should not be regarded as limiting.

Certain features of the invention that are, for clarity, described in the context of separate embodiments, may also be provided in combination in a single embodiment. Conversely, various features of the invention that are, for brevity, described in the context of a single embodiment, may also be provided separately or in any suitable sub-combination.

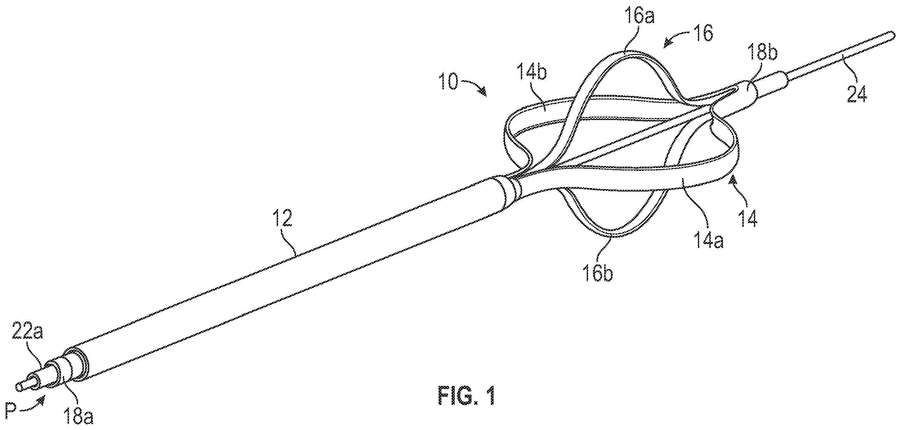

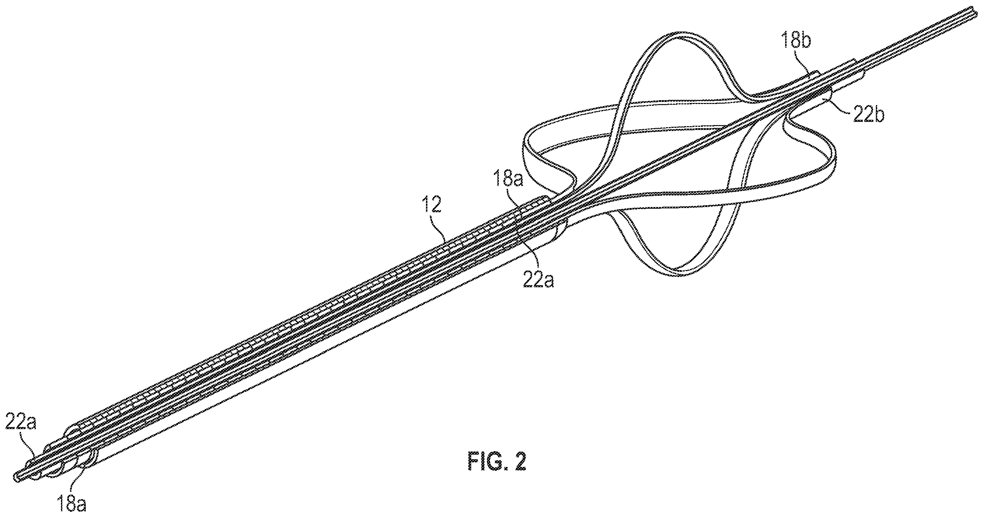

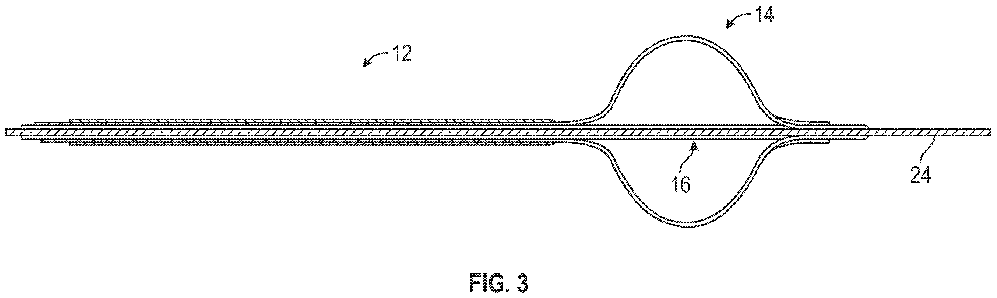

With reference to FIG. 1, a device is shown in the form of a catheter 10 including a tubular shaft 12 connected to at least two nested hoops, which include an outer hoop 14 and an inner hoop 16. As perhaps best shown in FIGS. 2, 3, and 4, the outer hoop 14 may comprise opposed, arcuate or outwardly bowed members 14a, 14b, which may comprise wires or ribbons, connected at their proximal and distal ends via proximal and distal outer tubular members (shafts) 18a, 18b. Similarly, the inner hoop 16 comprises opposed arcuate, outwardly bowed members 16a, 16b, which may also comprise wires or ribbons, connected at their proximal and distal ends via proximal and distal inner tubular members (shafts) 22a, 22b. As used in this disclosure, the term "hoop" connotes a structure generally in the form of a circle, but not necessarily a complete circle and possibly generally elliptical in shape (e.g., flattened and possibly connected to an intermediate structure, such as tubular members/shafts).

As can be appreciated, the diameter of the outer hoop 14 is greater than the diameter of the inner hoop 16. Similarly, the diameter of the tubular members 18a, 18b is greater than the diameter of the tubular members 22a, 22b, which are also nested when the device is assembled within the shaft 12. The arrangement is such that the outer and inner hoops 14, 16 are capable of rotating relative to each other, including by counter-rotating, when the proximal tubular members 18a, 22a are rotated. The proximal outer tubular member 18a may be coiled for enhanced flexibility, as shown, and the inner tubular members 22a, 22b may also optionally include a lumen for receiving a guidewire 24 for guiding the device to an endovascular location for providing a treatment, if desired. The proximal inner tubular member 22a may be solid to facilitate higher rotational speeds and torque transmission for the inner hoop 16.

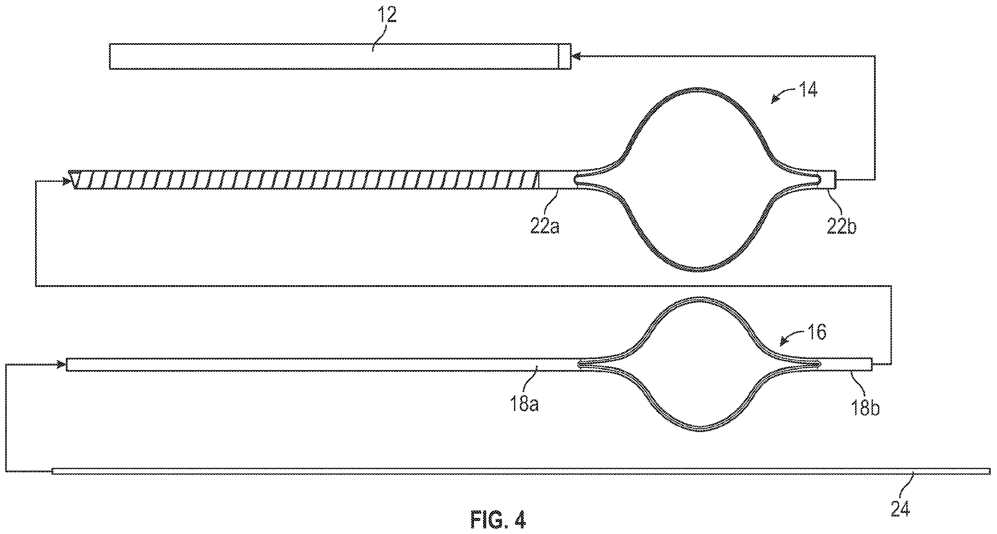

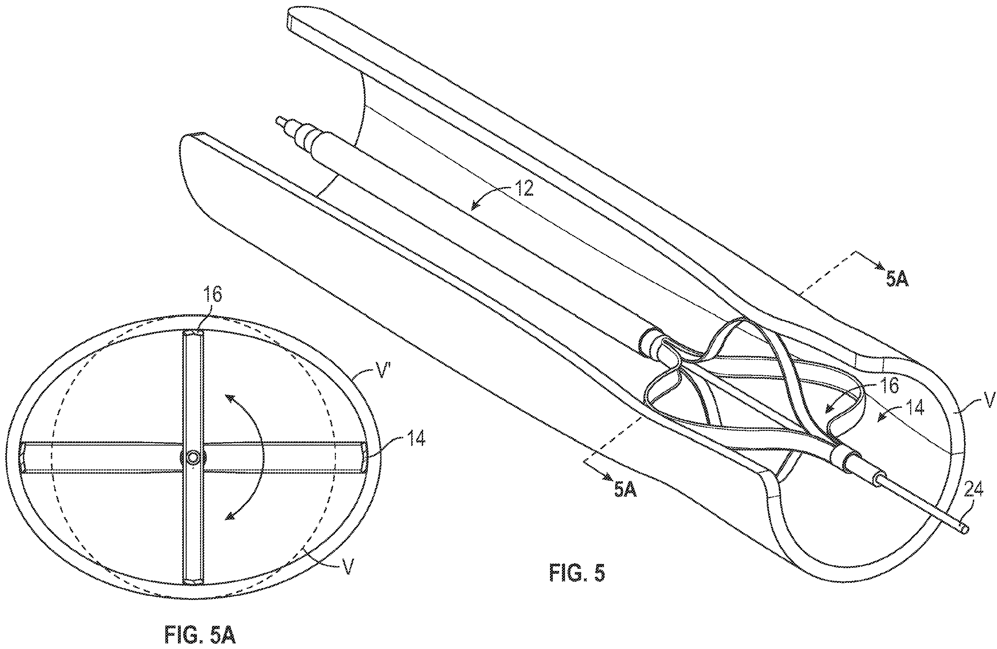

With reference now to FIGS. 5 and 5A, the device 10 may be positioned in a vessel V where treatment is desired, such as a location where ablation or debridement of the intima is needed. The diameter of the outer hoop 14 is selected such that it tends to cause local deformation of the vessel V along a plane aligned with the arcuate members 14a, 14b. As a result, as perhaps best shown in FIG. 4A, the local cross-section of the vessel V, which is normally generally circular, is caused to assume a more elliptical shape. When the inner hoop 16 is thus rotated, the members 20a, 20b engage the intima at locations spaced apart about 90 degrees from the locations where the arcuate members 14a, 14b are in engagement with the vessel. This engagement thus creates localized ablation or debridement of the intima.

By rotating the inner and outer hoops 14, 16 in a continuous manner, it can be appreciated that the endothelial layer of the vessel intima can be continuously treated. In particular, by causing the inner and outer hoops 14, 16 to counter-rotate, the deleterious effects of torsion may be eliminated, thus avoiding a corresponding decrease in efficacy. Because the inner hoop 16 makes direct contact with the corresponding portions of the intima when the vessel V is caused to assume the elliptical configuration, the phenomenon of only engaging tissue directly under a single cutter by simply rotating it in the vessel is avoided.

Relative rotation of the inner and outer hoops 14, 16 may be achieved manually by simply grasping and rotating the proximal tubular members 18a, 22a. Another approach is to connect these members 18a, 22a to corresponding motors to provide for the desired rotation. Typically, the relative rotation would be such that the inner hoop 16 is caused to rotate at a rotational speed faster than the rotational speed of the outer hoop, but the particular ratio used will depend on a variety of factors. Again, the relative rotation is capable of being in opposite directions.

In any case, compared to a single blade with one point of contact, the inner and outer hoops 14, 16 establish four points of contact with the intima of the vessel V. The effect of the edges of the nested hoops 14, 16 approaching each other during relative rotation allows for tissue to be dynamically sheared at double the rate of a single cutter. Consequently, the proposed device 10 could rotate less than a single bladed device (by as much as two to four times) to achieve the same ablating effect. Assuming rotational speeds equivalent to the current devices, then use of the proposed device would potentially result in half the time to perform a procedure.

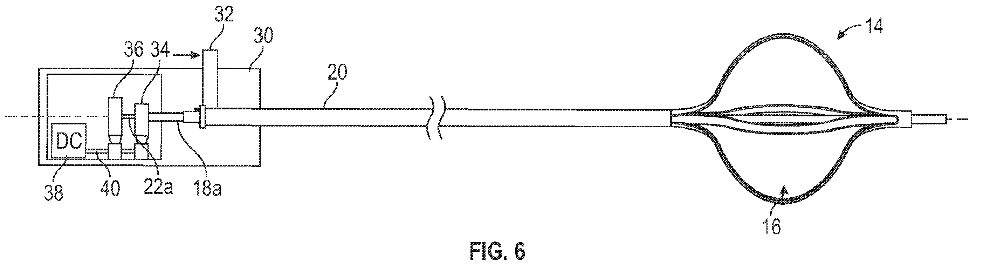

As shown in FIG. 6, the diameters of the outer and inner hoops 14, 16 may be fixed and passed through a suitable sheath 20 into the vessel. The sheath 20 may be made retractable and may be associated with a housing 30 connected to a proximal end of the device 10. A lever 32 may be provided for advancing or retracting the sheath 20 to expose or cover the hoops 14, 16.

The housing 30 may also include the motor(s) for rotating the hoops 14, 16. In the illustrated example, a single DC motor 38 is present, and connected to gears 34, 36 associated with the respective tubular members 18a, 22a to cause relative rotation. The direction and speed of rotation may be controlled by suitable gearing associated with an output shaft 40 from the motor 38, with the ultimate goal of achieving the desired treatment effect without causing excessive or undue levels of localized torsion (which again is largely avoided by using the option of counter-rotating hoops 14, 16).

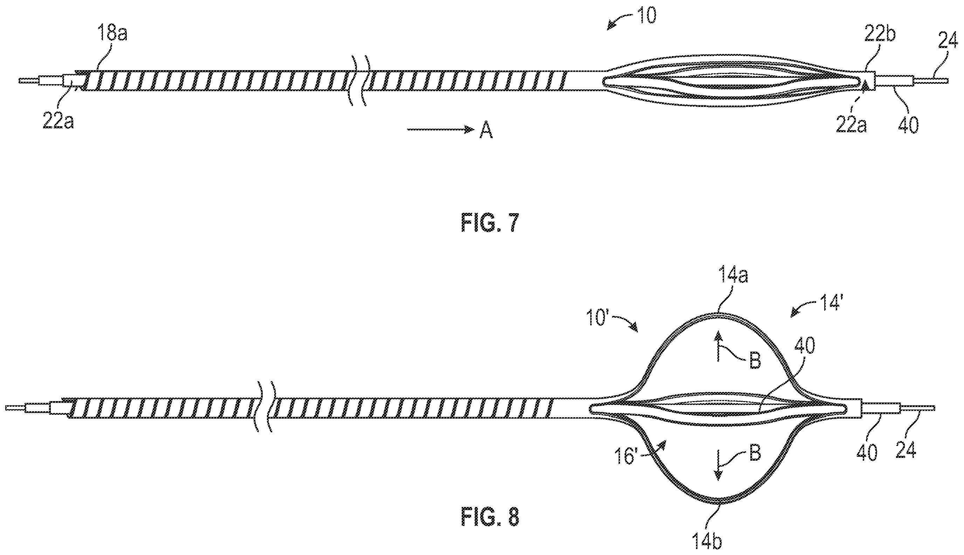

Alternatively, as shown in FIG. 7, the hoops 14, 16 of the device 10 may initially be retracted to a position generally parallel to and in substantial alignment with a longitudinal axis, and then the respective proximal tubular members 18a, 22a pushed longitudinally to achieve the desired diameter, as shown in FIG. 8. This may be achieved by including a further tubular member 40 to which the inner distal tubular member 18b is connected to fix their relative position longitudinally and rotationally, while the outer distal tubular member 22b couples to the inner member 18b for relative rotation. The tubular member 40 may also include a lumen for an optional guidewire 24, but could also be a solid material, such as a rod.

As can be appreciated, this arrangement allows for the advance (arrow A) of the tubular member 40 to cause the arcuate members 14a, 14b; 16a, 16b to deploy and bow outwardly (arrow B, 14' 16') in the desired manner to correspond to the diameter of or contact the vessel intima. This process may be done using imaging (e.g., radiographic/fluoroscopic guidance) and thus may allow for the device 10 to be customized *in situ* by the clinician in a dynamic fashion for achieving a desired result in vessel(s) having a variety of diameters. Similarly, by withdrawing the inner member 40, the arcuate members 14a, 14b; 16a, 16b to return to the retracted or home position, which may facilitate transport within the vessel, such as for advancing to a new treatment location or for withdrawal.

As can be appreciated, the proximal tubular members 18a, 22a may together form an annulus. This annulus may be sized for use in delivering a treatment agent, such as a drug, sclerosant, or the like, to adjacent the hoops 14, 16 and thus the treatment location. As indicated in FIG. 1, a delivery port P may be associated with the proximal ends of these tubular members 18a, 22a to allow for the introduction of such an agent. In the event a further tube or tubular shaft 40 is included as noted above, it may also serve as a means for delivery of a treatment agent via a port provided adjacent to the hoops 14, 16.

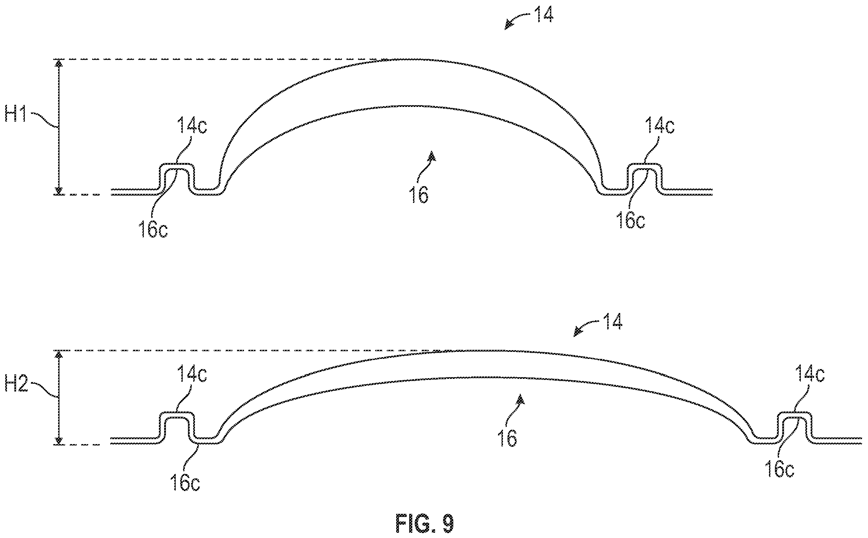

FIG. 9 schematically illustrates an alternative embodiment, in which a first portion, such as a radial projection 16c, of the inner hoop 16 is received within a second portion, such as a radial recess 14c, of the outer hoop 14. This may be done along the proximal and distal portions of the hoops 14, 16, as shown. The purpose of keying the hoops 14, 16 in this manner is to ensure proportional spacing remains as desired and to avoid collisions during relative rotation (compare dimensions H1 and H2, illustrating a larger and smaller diameters, respectively). This keying is especially useful where the hoops 14, 16 are formed of relatively flexible materials.

The hoops 14, 16 may be formed of a strong, yet flexible material, such as stainless steel. Shape memory materials, such as the Nitinol alloy, may also be used to achieve the desired degree of curvature. The leading and trailing portions of the inner hoop 16 may also be provided with sharpened edges to facilitate ablation and debridement of the endoluminal tissue.

The hoops 14, 16 may be separately formed and connected to the tubular members 18a, 18b; 22a, 22b, such as by welding or other forms of a secure fastening to permit the desired relative rotation, including at high speeds if necessary. Alternatively, the hoops 14, 16 may be formed by cutting opposed slits into a respective shaft, and then pressing the shaft ends toward each other. This causes the cut portions to bow outwardly, thereby forming the hoops 14, 16. As noted above, the relative diameter can also be adjusted in both cases by changing the degree of outward projection of the hoop(s) 14, 16.

As used herein, the following terms have the following meanings:
"A", "an", and "the" as used herein refers to both singular and plural referents unless the context clearly dictates otherwise. By way of example, "a compartment" refers to one or more than one compartment.

"About," "substantially," or "approximately," as used herein referring to a measurable value, such as a parameter, an amount, a temporal duration, and the like, is meant to encompass variations of +/- 20% or less, including +/-10% or less, +/-5% or less, +/-1% or less, and +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Although the invention has been described in conjunction with specific embodiments, many alternatives, modifications, and variations will be apparent to those skilled in the art. Accordingly, it embraces all such alternatives, modifications, and variations that fall within the scope of the appended claims. In addition, or identification of any reference in this application shall not be construed as an admission that such reference is available as prior art to the present disclosure.

## Claims

1. An apparatus for endovascular use for treating a vessel intima, comprising:
a catheter (10) adapted to form a plurality of nested hoops (14, 16) capable of rotating relative to one another in a deployed position, wherein the plurality of nested hoops (14, 16) comprise a first inner hoop (16) and a second outer hoop (14), the first inner hoop (16) having an outer diameter less than an inner diameter of the second outer hoop (14),
**characterized in that** the first inner hoop (16) and the second outer hoop (14) are capable of rotating in opposite directions relative to one another.

2. The apparatus of claim 1, wherein the catheter (10) comprises an inner tube (22a) connected to the first inner hoop (14) and an outer tube (18a) connected to the second outer hoop (14).

3. The apparatus of claim 2, wherein the outer tube (18a) is coiled.

4. The apparatus of claim 2, wherein the inner tube (22a) and the outer tube (18a) form an annulus for delivery of a treatment agent.

5. The apparatus of claim 2, wherein each of the inner hoop (14) and outer hoop (16) comprises arcuate members (14a, 14b, 16a, 16b) connected to the respective inner tube (22a) or outer tube (18a).

6. The apparatus of claim 2, wherein a distal end of the inner tube (22a) is rotatably connected to the outer tube (18a), and the inner tube (22a) is connected to a rod (40) for advancing distally to form the inner hoop (16) and outer hoop (14).

7. The apparatus of claim 1, further including one or more motors (38) adapted for causing relative rotation of the plurality of nested hoops (14, 16).

8. The apparatus of claim 1, further including one or more motors (38) adapted for causing counter-rotation of the plurality of nested hoops (14, 16).

9. The apparatus of claim 1, wherein the inner hoop (16) is partially received within a portion of the outer hoop (14).

## Patentansprüche

1. Einrichtung für eine endovaskuläre Verwendung zum Behandeln einer Gefäßintima, umfassend:
einen Katheter (10), der ausgebildet ist, um eine Vielzahl von verschachtelten Ringen (14, 16) auszubilden, die in der Lage sind, sich relativ zueinander in einer entfalteten Position zu drehen, wobei die Vielzahl von verschachtelten Ringen (14, 16) einen ersten Innenring (16) und einen zweiten Außenring (14) umfasst, wobei der erste Innenring (16) einen Außendurchmesser aufweist, der kleiner als ein Innendurchmesser des zweiten Außenrings (14) ist,
**dadurch gekennzeichnet, dass** der erste Innenring (16) und der zweite Außenring (14) in der Lage sind, sich relativ zueinander in entgegengesetzte Richtungen zu drehen.

2. Vorrichtung nach Anspruch 1, wobei der Katheter (10) einen Innenschlauch (22a), der mit dem ersten Innenring (14) verbunden ist, und einen Außenschlauch (18a) umfasst, der mit dem zweiten Außenring (14) verbunden ist.

3. Einrichtung nach Anspruch 2, wobei der Außenschlauch (18a) gewickelt ist.

4. Einrichtung nach Anspruch 2, wobei der Innenschlauch (22a) und der Außenschlauch (18a) einen Ringraum für eine Zuführung eines Behandlungsmittels ausbilden.

5. Einrichtung nach Anspruch 2, wobei jeder von dem Innenring (14) und dem Außenring (16) bogenförmige Elemente (14a, 14b, 16a, 16b) umfasst, die mit dem jeweiligen Innenschlauch (22a) oder Außenschlauch (18a) verbunden sind.

6. Einrichtung nach Anspruch 2, wobei ein distales Ende des Innenschlauchs (22a) mit dem Außenschlauch (18a) drehbar verbunden ist, und der Innenschlauch (22a) mit einem Stab (40) zum distalen Vorschieben verbunden ist, um den Innenring (16) und den Außenring (14) auszubilden.

7. Einrichtung nach Anspruch 1, ferner umfassend einen oder mehrere Motoren (38), die zum Bewirken einer Relativdrehung der Vielzahl von verschachtelten Ringen (14, 16) angepasst sind.

8. Einrichtung nach Anspruch 1, ferner umfassend einen oder mehrere Motoren (38), die zum Bewirken einer Gegendrehung der Vielzahl von verschachtelten Ringen (14, 16) angepasst sind.

9. Einrichtung nach Anspruch 1, wobei der Innenring (16) teilweise innerhalb eines Abschnitts des Außenrings (14) aufgenommen ist.

## Revendications

1. Appareil destiné à l'usage endovasculaire pour traiter l'intima d'un vaisseau, comprenant :
un cathéter (10) adapté pour former une pluralité de cerceaux emboîtés (14, 16) aptes à tourner les uns par rapport aux autres dans une position déployée, dans lequel la pluralité de cerceaux emboîtés (14, 16) comprend un premier cerceau interne (16) et une seconde boucle externe (14), le premier cerceau interne (16) ayant un diamètre externe inférieur à un diamètre interne du second cerceau externe (14),
**caractérisé en ce que** le premier cerceau interne (16) et le second cerceau externe (14) sont aptes à tourner en sens inverses l'un par rapport à l'autre.

2. Appareil selon la revendication 1, dans lequel le cathéter (10) comprend un tube interne (22a) relié au premier cerceau interne (14) et un tube externe (18a) relié au second cerceau externe (14).

3. Appareil selon la revendication 2, dans lequel le tube externe (18a) est enroulé.

4. Appareil selon la revendication 2, dans lequel le tube interne (22a) et le tube externe (18a) forment un espace annulaire destiné à l'administration d'un agent de traitement.

5. Appareil selon la revendication 2, dans lequel chacun de le cerceau interne (14) et du cerceau externe (16) comprend des éléments arqués (14a, 14b, 16a, 16b) reliés au tube interne (22a) ou au tube externe (18a) respectif.

6. Appareil selon la revendication 2, dans lequel une extrémité distale du tube interne (22a) est reliée de manière rotative au tube externe (18a), et le tube interne (22a) est relié à une tige (40) pour avancer de manière distale afin de former le cerceau interne (16) et le cerceau externe (14).

7. Appareil selon la revendication 1, comprenant en outre un ou plusieurs moteurs (38) aptes à provoquer une rotation relative de la pluralité de cerceaux emboîtés (14, 16).

8. Appareil selon la revendication 1, comprenant en outre un ou plusieurs moteurs (38) adaptés pour provoquer la contre-rotation de la pluralité de cerceaux emboîtés (14, 16).

9. Appareil selon la revendication 1, dans lequel le cerceau interne (16) est partiellement reçu dans une partie du cerceau externe (14).
